Europäisches Patentamt

European Patent Office

Office européen des brevets

(19)

(11) Veröffentlichungsnummer: **0 151 245**
**B1**

(12) **EUROPÄISCHE PATENTSCHRIFT**

(45) Veröffentlichungstag der Patentschrift: **26.09.90**

(21) Anmeldenummer: **84113937.1**

(22) Anmeldetag: **17.11.84**

(51) Int. Cl.⁵: **A 61 K 31/55** // (A61K31/55, 31:135)

(54) **Lorazepam-haltiges Schlafmittel.**

(30) Priorität: **08.02.84 DE 3404316**

(43) Veröffentlichungstag der Anmeldung:
**14.08.85 Patentblatt 85/33**

(45) Bekanntmachung des Hinweises auf die
Patenterteilung:
**26.09.90 Patentblatt 90/39**

(84) Benannte Vertragsstaaten:
**AT BE CH DE FR GB IT LI LU NL SE**

(56) Entgegenhaltungen:
DE-A-2 205 920

CHEMICAL ABSTRACTS, Band 83, Nr. 5, 4.
August 1975, Seite 49, Zusammenfassung Nr.
37765t, Columbus, Ohio, US; T. KLYGUL et al::

Die Akte enthält technische Angaben, die nach
dem Eingang der Anmeldung eingereicht
wurden und die nicht in dieser Patentschrift
enthalten sind.

(73) Patentinhaber: **MEDICHEMIE AG**
**Brühlstrasse 50**
**CH-4107 Ettingen/Basel (CH)**

(72) Erfinder: **Widauer, Josef Olaf, Dr.Med.**
**Beim Lindenbaum 17**
**CH-4123 Allschwil (CH)**

(74) Vertreter: **Lauer, Joachim, Dr.**
**Hug Interlizenz AG Austrasse 44 Postfach**
**CH-8045 Zürich (CH)**

(56) References cited:
"Experimental characteristics of the
pharmacological activity spectrum of the
tranquilizer Lorazepam", & ZH. NEVROPATOL.
PSIKHIATR. IM. S. S. KORSAKOVA 1975, 75(3),
435-8

**Beschreibung**

Die vorliegende Erfindung betrifft ein Lorazepam-haltiges Schlafmittel für orale oder enterale Verabreichung.

Für viele insbesondere kranke Menschen ist die Erfüllung des vitalen Bedürfnisses nach einem erholsamen Schlaf letzlich nur mit medikamentöser Nachhilfe möglich. Dies ist seit vielen Jahrzehnten ein Ansporn zur Neu- und Weiterentwicklung noch besser geeigneter Schlafmittel. Unter diesen nehmen die Benzodiazepin-Hypnotika einen breiten Raum ein. Vertreter dieser Gruppe mit langer Halbwertszeit führen nach wiederholter Anwendung zur Kumulation und zum morgendlichen Hangover während Benzodiazepine mit sehr kurzer Halbwertszeit zu Absetzeffekten und einem erhöhten Abhängigkeitpotential neigen. In dieser Gruppe von Hypnotika ist Lorazepam mit einer mittleren Halbwertszeit ein wichtiger Vertreter. Aus der langjährigen Anwendung dieser Substanz ist jedoch bekannt, dass der Einsatz als Hypnotikum nicht unproblematisch ist, da bei Gabe etwas höherer Dosen zum Teil relativ starke direkte Nebenwirkungen wie Muskelrelaxation, Ataxie und sedative Nachwirkungen in den nächsten Tag hinein auftreten können. Diese Nebenwirkungen sind insbesondere bei älteren Patienten oft bereits bei Einzeldosen von 2—3 mg verstärkt zu sehen, so dass Einzeldosen von mehr als 1 mg Lorazepam vom Gesichtspunkt des Nebenwirkungsrisikos problematisch sein können.

Bisher ist es nicht gelungen, die Wirkung von Lorazepam zu verstärken, ohne, dabei die genannten Nebenwirkungen in Kauf nehmen zu müssen.

In der deutschen Patentschrift 2.205.920 ist insbesondere das Kombinationspräparat, bestehend aus 10 Teilen des starken Hypnotikums/Sedativums Aethchlorvynol und 1 Teil Diphenhydramin beschrieben. Durch diese Kombination werden die sedierenden Eigenschaften des Aethchlorvynols nur geringfügig verändert, dagegen wird die Toxizität vermindert.

In Chemical Abstracts *83*:37765t (1975) wird gezeigt, dass Lorazepam stärkere transquillierende und sedative Wirkungen aufweist als die verwandten Azepame Diazepam (Valium), Nitrazepam (Mogadon) und Chlordiazepoxid (Librium) und dass dessen hypnotische Wirkung etwa gleich stark ist wie die des Diazepams. Lorazepam wirkt in erster Linie transquillierend und sedativ und erst in zweiter Linie schlafmachend (hypnotisch). Bei der Verwendung von Lorazepam in Schlafmitteln war daher a priori mit starken sedativen Nebenwirkungen zu rechnen.

Darüber hinaus enthält diese Literaturrstelle keine Lehre zur Entwicklung eines Schlafmittels, das sich durch weitgehendes Fehlen von Nebenwirkungen wie Muskelrelaxation oder hangover auszeichnet.

Es besteht daher ein Bedürfnis nach einem wirksamen und gleichzeitig gut verträglichen Schlafmittel. Dieses Ziel lässt sich erfahrungsgemäss durch Erhöhung der Lorazepam-Dosis schlecht erreichen.

Der Erfindung liegt die Aufgabe zugrunde, dem Arzt ein wirksames aber nur relativ geringe Mengen von Lorazepam enthaltendes Schlafmittel in die Hand zu geben, dessen Anwendung keine der genannten Nebenwirkungen zur Folge hat.

Es wurde gefunden, dass sich die hypnotische Wirkung von Lorazepam durch Kombination mit Diphenhydramin entscheidend verstärken lässt, ohne dabei die zu erwartende additive Toxizität, die sich aus der Summe der Toxizitäten der Komponenten ergibt, in Kauf nehmen zu müssen.

Bei oraler Verabreichung an Mäuse beträgt die akute Toxizität DL 50 von Diphenhydramin-hydrochlorid 312 mg/kg, die von Lorazepam 3178 mg/kg.

Eine Kombination aus 25 Gewichtsteilen Diphenhydramin-hydrochlorid und einem Gewichtsteil Lorazepam müsste demnach additiv gerechnet eine DL 50 von 323,2 mg/kg aufweisen.

Berechnung:

$$25\ \frac{1}{312\ \text{mg/kg}} + 1\ \frac{1}{3178} = \frac{26}{x}\ ;\quad x = 323.2\ \text{mg/kg}$$

Diphenhydr.     Loraz.    DL 50 Komb.

Ueberraschenderweise hat die tierexperimentelle toxikologische Prüfung der Kombination aus einem Gewichtsteil Lorazepam und 25 Gewichtsteilen Diphenhydramin-hydrochlorid im Vergleich zur toxikologischen Prüfung von Diphenhydramin allein eine progrediente Abnahme der akuten Giftigkeit bei steigenden Dosen ergeben.

Tabelle 1 siehe Seite 4.

So liegt nach oraler Verabreichung an männlichen Mäusen die DL 50 der genannten Kombination bei 430 mg/kg. Bei weiteren Dosissteigerungen über die DL 50 hinaus, nimmt die Toxizität der Kombination gegenüber derjenigen von reinem Diphenhydramin zunehmend ab. So ergibt sich z.B. für Diphenhydramin eine DL 95 von 645 mg/kg, für die Kombination beträgt diese jedoch nur 1622 mg/kg, d.h. die Kombination ist bei weitem besser verträglich als Diphenhydramin.

Aus diesem Befund ist auch zu schliessen, dass sich die in der Kombination enthaltenen pharmakologisch aktiven Komponenten bei verstärkter hypnotischer Wirkung im therapeutischen Dosenbereich in ihrer akuten Giftigkeit — unter Zugrundlegund der Toxizität von Diphenhydramin — antagonisieren.

Dieser tierexperimentelle Befund kann im Hinblick auf eine in suizidaler Absicht erfolgten Einnahme hoher Dosen eine zusätzliche Sicherheit dieses neuen Schlafmittels beim Menschen bieten.

TABELLE 1

Akute orale Toxizität an weissen Mäusen von reinem Diphenhydramin·HCl und des Kombinationspräparates (Komb.) aus 25 Gewichtsteilen Diphenhydramin·HCl und 1 Gewichtsteil Lorazepam.

| Lethalität DL 5 - 95 | Dosen in mg/kg Körpergewicht | | | |
|---|---|---|---|---|
| | Diph.·HCl | | Kombination | |
| | männl. | weibl. | männl. | weibl. |
| 05 | 151 | 153 | 114 | 171 |
| 10 | 181 | 187 | 159 | 220 |
| 15 | 203 | 212 | 196 | 258 |
| 20 | 221 | 233 | 230 | 291 |
| 25 | 238 | 252 | 262 | 321 |
| 30 | 253 | 269 | 293 | 349 |
| 35 | 268 | 286 | 325 | 378 |
| 40 | 282 | 303 | 358 | 406 |
| 45 | 297 | 320 | 392 | 436 |
| 50 | 312 | 338 | 430 | 466 |
| 55 | 328 | 356 | 470 | 500 |
| 60 | 345 | 376 | 516 | 536 |
| 65 | 363 | 398 | 568 | 576 |
| 70 | 384 | 424 | 630 | 623 |
| 75 | 409 | 453 | 705 | 679 |
| 80 | 439 | 489 | 803 | 749 |
| 85 | 478 | 537 | 940 | 843 |
| 90 | 536 | 608 | 1158 | 987 |
| 95 | 645 | 742 | 1622 | 1274 |

Erste klinische Versuche haben gezeigt, dass das Kombinationspräparat aus Lorazepam und Diphenhydramin die gewünschte Wirkungssteigerung gegenüber reinem Lorazepam aufweist, ohne dass diese durch verstärkte Nebenwirkungen erkauft werden musste. Im Gegenteil die Zahl und Stärke von Nebenwirkungen wurden eher vermindert. Geprüft wurde die Wirkung des Kombinationspräparates aus 1 mg Lorazepam und 25 mg Diphenhydramin-hydrochlorid in Tablettenform. Dieses Schlafmittel wurde ärztlich betreuten Patienten eines Altersheims sowie Patienten einer ärztlichen Allgemeinpraxis in Fällen von deutlichen Ein- und Durchschlafstörungen verschiedener Ursache und Stärke abgegeben und hinsichtlich seiner Wirkung und Verträglichkeit geprüft. Die Patienten erheilten vor dem Zubettgehen jeweils eine Tablette mit etwas Flüssigkeit. Patienten oder Pflegepersonal führten Buch über die wichtigsten Parameter. Die folgenden Daten wurden ermittelt:

3

Tabelle 2                ALTERSVERTEILUNG

| Alter | Patientenzahl |
|---|---|
| unter 20 Jahren | 8 |
| 20 - 29 Jahren | 16 |
| 30 - 39 Jahren | 25 |
| 40 - 49 Jahren | 28 |
| 50 - 59 Jahren | 22 |
| 60 - 69 Jahren | 26 |
| 70 - 80 Jahren | 31 |

Tabelle 3                ART DER SCHLAFSTOERUNGEN

| Art der Schlafstörungen | Patientenzahl | % des Gesamtkollektivs |
|---|---|---|
| Einschlafstörungen | 42 | 27 |
| Durchschlafstörungen | 39 | 25 |
| Ein- + Durchschlafst. | 75 | 48 |

Tabelle 4                EINSCHLAFZEIT

| Einschlafzeiten | Patientenzahl | % des Gesamtkollektivs |
|---|---|---|
| bis   10 Min. | 12 | 7,7 |
| 20 Min. | 57 | 36,6 |
| 30 Min. | 51 | 32,7 |
| 40 Min. | 21 | 13,4 |
| 60 Min. | 9 | 5,8 |
| über  60 Min. | 6 | 3,8 |

Tabelle 5                SCHLAFDAUER

| Schlafdauer | Patientenzahl | % des Gesamtkollektivs |
|---|---|---|
| bis  4 Stunden | 21 | 13,4 |
| 4  - 6 Stunden | 36 | 23 |
| 6  - 8 Stunden | 79 | 51 |
| über 8 Stunden | 20 | 12,6 |

112 von 156 Patienten beziechneten die therapeutische Wirkung als gut bis sehr gut. Sie schliefen nachts in der Regel mehr als 6 Stunden gleichmässig durch un fühlten sich am Morgen frisch und erholt. Es traten dabei keine Nebenwirkungen auf.

24 Patienten beurteilten die Wirkung als ausreichend. Sie schliefen mehr als 4 Stunden durch.

19 Patienten beurteilten die Wirkung als nicht ausreichend. Die meisten dieser Gruppe litten an Verstimmungszuständen depressiver Art.

Die Gruppe der 92 Patienten, die bereits vorher Schlafmittel zu sich nahmen, beschrieben im subjektiven Vergleich das neue Präparat als besser oder mindestens ebenbürtig.

Die allgemeine Verträglichkeit des Kombinationspräparates kann als sehr gut beurteilt werden. Patienten, die über Schwindel oder gelegentliche Kopfschmerzen klagten, hatten in der Regel diese Beschwerden auch schon früher vorgebracht.

Aufgrund der bisher vorliegenden Erfahrungen hat sich das neue Kombinationspräparat als gut wirksames und sehr gut verträgliches Schlafmittel mit breitem Anwendungsspektrum erwiesen. Gemäss der niederen Dosierung der Einzelkomponenten hätte erwartet werden können, dass in erster Linie nur die leichteren, emotionell bedingten Einschlafstörungen auf die Behandlung ansprechen würden. Auffallenderweise liessen sich aber auch Durchschlafstörungen sowie Schlafstörungen in höheren Alter verschiedner Genese sehr günstig beeinflussen, ohne dass es zue ernsthaften Nebenwirkungen oder zu paradoxen Reaktionen gekommen ist. Der Vergleich mit früher eingenommenen Schlafmitteln fiel meistens zugunsten des Versuchspräparates aus.

Gegenstand der Erfindung ist eine Lorazepam-haltiges Schlafmittel zu oralen oder enteralen Verabreichung, dadurch gekennzeichnet, das es

    a) Lorazepam und

    b) Diphenhydramin oder ein Säureadditionssalz von Diphenhydramin im Gewichtsverhältnis von 1:10 bis 1:75 enthält.

Als besonders zweckmässig hat sich das Gewichtsverhältnis 1:20 bis 1:35 erwiesen.

Ein weiterer Gegenstand der Erfindung ist das Verfahren zur Herstellung eines Lorazepam-haltigen Schlafmittels, das dadurch gekennzeichnet ist, dass man Lorazepam mit Diphenhydramin oder einem Säureadditionssalz von Diphenhydramin in einem Gewichtsverhältnis von 1:10 bis 1:75 mischt und die erhaltene Komposition zur oralen oder enteralen Darreichungsformen verarbeitet.

Die Mischung erfolgt zweckmässig im Gewichtsverhältnis 1:20 bis 1:35.

Lorazepam ist der nicht wortgeschützte Freiname (Generic name) von 7-Chlor-5-(2-chlorphenyl)-1,3-dihydro-3-hydroxy-2H-1,4-benzodiazepin-2-on.

Diphenhydramin ist der Freiname für 2-(Diphenylmethoxy)-N,N-dimethylethanamin.

Als Säuren für die Bildung therapeutisch anwendbarer Säureadditionssalze von Diphenhydramin eignen sich im Prinzip alle Säuren, die gut verträgliche Salze bilden wie z.B. Salzsäure, Phosphorsäure, Salizylsäure, Acetylsalizylsäure, Weinsäure, Ascorbinsäure, 4-Sulfamidobenzosäure usw. Gewöhnlich wird das Diphenhydramin hydrochlorid oder die freie Base verwendet. Die andern Säureadditionssalze sind jedoch im allgemeinen ebensogut geeignet wie das Hydrochlorid. Je nachdem was für ein Salz von Diphenhydramin gewählt wird, verschiebt sich das optimale Mischungsverhältnis zwischen Lorazepam und dem Diphenhydraminsalz entsprechend dem veränderten Molekulargewicht dieses Salzes.

Als orale Darreichungsformen für das Kombinationspräparat aus Lorazepam und Diphenhydramin kommen in Betracht Tabletten, Dragées, Kapseln, Pulver und Suspensionen.

Geeignete enterale Darreichungsformen sind Suppositorien oder Klistiere.

Feste orale Darreichungsformen werden im allgemeinen bevorzugt.

Die Dosierung der beiden Wirkstoffe ergibt sich aus deren Aktivitäten.

Langjährige Erfahrungen mit Lorazepam als Hypnotikum zeigen, dass eine Einzeldosis von 1 mg die Einnahmemenge darstellt, welche ein besonders günstiges Verhältnis von Wirkung und Verträglichkeit aufweist. Die hypnotische Wirkung von 1 mg Lorazepam ist jedoch häufig unzureichend. Die Dosierung wird daher oft auf 2—3 mg gesteigert, wobei dann in einzelnen Fällen subjektiv unangenehme Nebenwirkungen auftreten können.

Gaben von 25 mg Diphenhydramin (als Base oder Säureadditionssalz) haben gegenüber Placebo bereits einen hoch signifikanten hypnotischsedativen Effekt, der bei Erhöhung au die übliche hypnotische Dosis von 50—75 mg, nicht mehr wesentlich zunimmt. Der geringe Wirkungszuwachs muss zudem mit spürbaren Nebenwirkungen wie trockener Mund, Harnverhalten und erhöhter Augendruck erkauft werden. Daraus ergibt sich für das Kombinationspräparat eine Spanne reichend von 1—3 Gewichtsteilen Lorazepam und 25—75 Gewichtsteilen Diphenhydramin bzw. Diphenhydramin-Säureadditionssalz.

Als besonders zweckmässig und ausreichend haben sich Dosen von 1 Gewichtsteil Lorazepam und 20—35 Gewichtsteilen Diphenhydramin bzw. Diphenhydramin-Säureadditionssalz erwiesen.

Einzeldosen enthalten 1—3 mg Lorazepam und etwa 25—100 mg Diphenhydramin bzw. ein Salz davon.

Bevorzugte Einzeldosen enthalten gewöhnlich 1 mg Lorazepam und 20—35 mg Diphenhydramin oder ein Salz davon. Solche Dosen haben eine ausgesprochen geringe Inzidenz zu substanzspezifischen Nebenerscheinungen. Sie stellen also bezüglich der Verträglichkeit eine minimale Belastung dar. Auf der andern Seite ist ihre Wirkung für die meisten praktisch auftretenden Fälle völlig ausreichend. In der Regel werden den festen Darreichungsformen ausser den beiden Wirksubstanzen noch ungiftige pharmazeutische Trägersubstanzen wie Maisstärke, Weizenstärke, Talk, Natriumbicarbonat, Weinsäure, Zitronensäure, Magnesiumstearat usw. zugesetzt. Die Mischung aus den Wirkstoffen und den Zusätzen wird in der Regel zu Tabletten gepresst. Sie können nötigenfalls anschliessend noch dragiert werden. Besonders wenn sie für die Pädiatrie bestimmt sind.

Für die Herstellung von Suppositorien wird die Wirkstoffmischung mit den Trägersubstanzen und Suppositorienmasse vermischt, in der Suppositorienmasse geschmolzen und zu Suppositorien vergossen oder gepresst.

Flüssige Darreichungsformen sind in der Regel wässerige Lösungen von Diphenhydramin-Salzen, in denen Lorazepam suspendiert ist. Zur Stabilisierung der Suspensionen werden Netzmittel oder Polyhole wie z.B. Polyethylenglykol oder Polypropylenglykol sowie Glykol oder Glycerinester zugesetzt.

## Beispiel 1

Tabletten

2,5 kg Diphenhydramin·Hydrochlorid und 100 g Lorazepam werden mit Mais- oder Kartoffelstärke, Milchzucker, Zellulosepulver, Magnesiumstearat und Talk zu einem Granulat verarbeitet und zu 100,000 Tabletten mit einem Gehalt von je 1 mg Lorazepam und 25 mg Diphenhydramin·Hydrochlorid gepresst.

## Beispiel 2

Dragées

Nach Beispiel 1 hergestellte Tabletten werden noch mit einem Zuckerüberzug dragiert.

## Beispiel 3

Tabletten

Nach Beispiel 1 hergestellte Tabletten werden mit einem Film überzogen, der aus Polymeren wie Zellulosederivaten, Polymethacrylsäureester, Polyvinylpyrrolidon, Polyethylenglykol besteht.

## Beispiel 4

Tabletten

6,47 kg Diphenhydramin·Salizylat werden mit 100 g Lorazepam vermischt, ähnlich wie im Beispiel 1 beschrieben, granuliert und danach zu 200,000 Tabletten gepresst.

## Beispiel 5

Kapseln

2,5 Diphenhydramin·Hydrochlorid und 0,1 kg Lorazepam werden mit den üblichen Zusätzen, wie unter Beispiel 1 erwähnt, vermischt und direkt oder nach vorherigem Granulieren in 100,000 Hartgelatine Kapseln mit einem Gehalt von je 1 mg Lorazepam und 25 mg Diphenhydramin·Hydrochlorid abgefüllt.

## Beispiel 6

Suppositorien

2,5 kg fein pulverisiertes Diphenhydramin·Hydrochlorid und 0,1 kg fein pulverisiertes Lorazepam werden mit Suppositorienmasse, die zur Hauptsache aus hydrierten pflanzlichen Fetten besteht, vermischt und zu 100,000 Suppositorien vergossen mit einem Gehalt von je 1 mg Lorazepam und 25 mg Diphenhydramin·Hydrochlorid.

**Patentansprüche**

1. Lorazepam-haltiges Schlafmittel zur oralen oder enteralen Verabreichung, dadurch gekennzeichnet, dass es
a) Lorazepam und
b) Diphenhydramin oder ein Säureadditionssalz von Diphenhydramin im Gewichtsverhältnis von 1;10 bis 1:75 enthält.

2. Mittel nach Patentanspruch 1, dadurch gekennzeichnet, dass das Gewichtsverhältnis 1:20 bis 1:35 beträgt.

3. Verfahren zur Herstellung eines Lorazepam-haltigen Schlafmittels, dadurch gekennzeichnet, dass man Lorazepam mit Diphenhydramin oder einem Säureadditionssalz von Diphenhydramin in einem Gewichtsverhältnis von 1:10 bis 1:75 mischt und die erhaltene Komposition zur oralen oder enteralen Darreichungsformen verarbeitet.

4. Verfahren nach Patentanspruch 3, dadurch gekennzeichnet, dass man die Komponenten in einem Gewichtsverhältnis von 1:20 bis 1:35 mischt.

**Revendications**

1. Somnifère contenant du lorazépam pour administration orale ou entérale, caractérisé en ce qu'il renferme
a) du lorazépam, et

b) de la diphénhydramine ou un sel d'addition d'acide de la diphénhydramine, dans un rapport pondéral de 1/10 à 1/75.

2. Médicament selon la revendication 1, caractérisé en ce que le rapport pondéral est de 1/20 à 1/35.

3. Procédé de préparation d'un somnifère contenant du lorazépam, caractérisé en ce que l'on mélange le lorazépam avec la diphénhydramine ou un sel d'addition d'acide de la diphénhydramine dans un rapport pondéral de 1/10 à 1/75, et que l'on transforme la composition obtenue en des formes d'administration orale ou entérale.

4. Procédé selon la revendication 3, caractérisé en ce que l'on mélange les composants dans une proportion pondérale de 1/20 à 1/35.

**Claims**

1. Lorazepam-containing soporific agent for oral or enteral administration, characterized in that it contains

a) lorazepam and

b) diphenhydramine or an acid addition salt of diphenhydramine in a weight ratio of 1:10 to 1:75.

2. Agent according to Patent Claim 1, characterized in that the weight ratio is 1:20 to 1:35.

3. Process for the preparation of a lorazepam-containing soporific agent, characterized in that lorazepam is mixed with diphenhydramine or an acid addition salt of diphenhydramine in a weight ratio of 1:10 to 1:75 and the resulting composition is processed to oral or enteral presentation forms.

4. Process according to Claim 3, characterized in that the components are mixed in a weight ratio of 1:20 to 1:35.